# EUROPEAN PATENT APPLICATION

(11) **EP 4 406 561 A1**
(43) Date of publication of application: **31.07.2024**
(21) Application number: 22944032.6
(22) Date of filing: 29.11.2022
(51) Int. Cl.: A61L 9/01

(54) **METHOD AND DEVICE FOR INACTIVATING NOVEL CORONAVIRUS**

(71) Applicant: Yokota Trading Co. Ltd., Hiroshima-shi, Hiroshima 730-0051 (JP)
(72) Inventor: TAGAWA, Masatake, Hiroshima-shi Hiroshima 730-0051 (JP)
(74) Representative: Maikowski & Ninnemann Patentanwälte Partnerschaft mbB
(86) International application number: PCT/JP2022/043971
(87) International publication number: WO 2024/116279

(57) **Abstract**

Provided are a new coronavirus inactivation method and a new coronavirus inactivation device that are capable of quick inactivation, are safe, and are practical.

The new coronavirus inactivation device is a device for inactivating new coronavirus SARS-CoV-2 by singlet oxygen, the device including: a contactor configured to bring an aqueous rose bengal solution and air containing the new coronavirus SARS-CoV-2 into contact with each other in a state where light is applied to the aqueous rose bengal solution, wherein the aqueous rose bengal solution has a concentration of not less than 10 µM and not greater than 50 µM, and the light applied to the aqueous rose bengal solution has an illuminance of not less than 500 lux and not greater than 8000 lux.

## Description

### BACKGROUND OF THE INVENTION

### (Field of the Invention)

The present invention relates to a method and device for inactivating a new coronavirus. In the present specification and the claims, the concentration of an aqueous rose bengal solution means the concentration of rose bengal in the aqueous rose bengal solution. In addition, a unit M representing the concentration is mol/L, and 10 µM is 10 µmol/L.

### Background Technology

### (Description of Related Art)

In recent years, the new coronavirus SARS-CoV-2 has been raging around the world. In response to this situation, efforts have been made to inactivate the new coronavirus SARS-CoV-2.

For example, a method for inactivating the new coronavirus SARS-CoV-2 using a photocatalyst has been proposed. This method inactivates the new coronavirus SARS-CoV-2 using active oxygen generated by irradiating photocatalysts with excitation light, and experimental data of inactivation of the new coronavirus SARS-CoV-2 in liquid and the new coronavirus SARS-CoV-2 in aerosol are also disclosed.

Another method for inactivating the virus is to inactivate the virus by singlet oxygen generated by applying light to a photosensitizing dye (see, for example, Patent Document 1). Patent Document 1 indicates that a virus inactivation method using singlet oxygen is effective for enveloped viruses and non-enveloped viruses. The new coronavirus SARS-CoV-2 is an enveloped virus and can therefore be expected to be inactivated by singlet oxygen.

### [Related Document]

### [Patent Document]

[Patent Document 1] Japanese Laid-Open Patent Publication No.

### SUMMARY OF THE INVENTION

As a device for inactivating the new coronavirus SARS-CoV-2 in the air, a device equipped with an active oxygen generator using a photocatalyst or a singlet oxygen generator using a photosensitizing dye is conceivable by referring to the above findings. However, there are many matters to be considered in order to make such a new coronavirus inactivation device into a practical device.

In order to put the new coronavirus inactivation device into practical use, it is important to inactivate the new coronavirus SARS-CoV-2 quickly, and this also requires setting of appropriate operating conditions and some device contrivances. In order to make the new coronavirus inactivation device into a practical device, it is also important to be able to provide devices having various sizes, from small to large, in response to the location of use, and to be able to use the device continuously over a long period of time, for example. It is also essential for practical use that the new coronavirus inactivation device be safe.

An object of the present invention is to provide a new coronavirus inactivation method and a new coronavirus inactivation device that are capable of quick inactivation, are safe, and are practical.

The present invention is directed to a new coronavirus inactivation method for bringing an aqueous rose bengal solution and air containing new coronavirus SARS-CoV-2 into contact with each other in a state where light is applied to the aqueous rose bengal solution, and inactivating the new coronavirus SARS-CoV-2 by generated singlet oxygen, wherein the aqueous rose bengal solution has a concentration of not less than 10 µM and not greater than 50 µM, and the light applied to the aqueous rose bengal solution has an illuminance of not less than 500 lux and not greater than 8000 lux.

The present invention is also directed to a new coronavirus inactivation device for inactivating new coronavirus SARS-CoV-2 by singlet oxygen, the new coronavirus inactivation device including: a contactor configured to bring an aqueous rose bengal solution and air containing the new coronavirus SARS-CoV-2 into contact with each other in a state where light is applied to the aqueous rose bengal solution, wherein the aqueous rose bengal solution has a concentration of not less than 10 µM and not greater than 50 µM, and the light applied to the aqueous rose bengal solution has an illuminance of not less than 500 lux and not greater than 8000 lux.

In the new coronavirus inactivation device according to the present invention, the contactor is configured to blow the air containing the new coronavirus SARS-CoV-2 into the aqueous rose bengal solution to which the light is applied.

In the new coronavirus inactivation device according to the present invention, the contactor configured to blow the air includes a jet nozzle or jet hole through which the air containing the new coronavirus SARS-CoV-2 is blown into the aqueous rose bengal solution to which the light is applied.

The new coronavirus inactivation device according to the present invention further includes a concentration adjusting device including a sensor configured to detect the concentration of the aqueous rose bengal solution and a supplier configured to supply rose bengal powder or a high-concentration aqueous rose bengal solution, the concentration adjusting device being configured to adjust the concentration of the aqueous rose bengal solution into a set range.

In the new coronavirus inactivation device according to the present invention, the concentration adjusting device detects a color of the aqueous rose bengal solution by the sensor and adjusts the concentration of the aqueous rose bengal solution into the set range by using a correlation between the color and the concentration of the aqueous rose bengal solution.

The new coronavirus inactivation device according to the present invention further includes a water level adjusting device configured to keep a water level of the aqueous rose bengal solution at a set water level.

According to the present invention, it is possible to provide a new coronavirus inactivation method and a new coronavirus inactivation device that are capable of quick inactivation, are safe, and are practical.

### BRIEF DESCRIPTION OF THE DRAWINGS

Fig. 1 illustrates the configuration of a new coronavirus inactivation device 1 of a first embodiment of the present invention;
Fig. 2 illustrates the configuration of a new coronavirus inactivation device 3 of a second embodiment of the present invention;
Fig. 3 illustrates the configurations of new coronavirus inactivation devices 4 and 5 of a third embodiment of the present invention;
Fig. 4 illustrates the configuration of a new coronavirus inactivation device 6 of a fourth embodiment of the present invention;
Fig. 5 illustrates the configuration of a new coronavirus inactivation device 7 of a fifth embodiment of the present invention;
Fig. 6 illustrates the configuration of a test apparatus 150 of Example 1 of the present invention; and
Fig. 7 is a photograph showing the relationship between the concentration and the color of an aqueous rose bengal solution.

### DESCRIPTION OF EMBODIMENTS

The new coronavirus inactivation method of the present invention is a method for inactivating the new coronavirus SARS-CoV-2 in the air by singlet oxygen generated by using rose bengal. When light having a predetermined wavelength is applied to an aqueous rose bengal solution, the rose bengal becomes excited, and when the air comes into contact with the excited rose bengal, the oxygen in the air is excited and becomes singlet oxygen. This singlet oxygen inactivates the new coronavirus SARS-CoV-2 in the air.

Specifically, in the new coronavirus inactivation method of the present invention, an aqueous rose bengal solution and the air containing the new coronavirus SARS-CoV-2 are brought into contact with each other in a state where light is applied to the aqueous rose bengal solution, and the new coronavirus SARS-CoV-2 is inactivated by generated singlet oxygen. The concentration of the aqueous rose bengal solution used here is preferably not less than 10 µM and not greater than 50 µM, and the illuminance of the light applied to the aqueous rose bengal solution is preferably not less than 500 lux and not greater than 8000 lux.

The aqueous rose bengal solution used here is obtained by dissolving known rose bengal, which is also used as a food coloring agent, in water. The water included in the aqueous rose bengal solution is also not particularly limited. Since rose bengal is easily dissolved in water, aqueous rose bengal solutions having a wide range of concentrations can be prepared.

The concentration of the aqueous rose bengal solution is preferably not less than 10 µM. As shown in Example 2 described later, in the case where the concentration of the aqueous rose bengal solution was 10 µM and the illuminance of the light applied to the aqueous rose bengal solution was 600 lux, the new coronavirus SARS-CoV-2 was inactivated within a very short time (within a few seconds). As can be seen from this, if the concentration of the aqueous rose bengal solution is about 10 µM, the new coronavirus SARS-CoV-2 can be inactivated within a very short time. If the concentration of the aqueous rose bengal solution is not greater than 10 µM, the speed of inactivation of the new coronavirus SARS-CoV-2 cannot be considered sufficient for practical use. The upper limit of the concentration of the aqueous rose bengal solution is about 50 µM for practical use, considering the speed of inactivation of the new coronavirus SARS-CoV-2 and economic efficiency.

The light applied to the aqueous rose bengal solution or with which the aqueous rose bengal solution is irradiated is used to bring the rose bengal into an excited state and excite the oxygen in the air to generate singlet oxygen, and thus light having a wavelength suitable for this is needed. The maximum absorption wavelength of rose bengal is 549 nm, and the excitation wavelength of rose bengal is 537 nm, so that the light applied to the aqueous rose bengal solution or the light with which the aqueous rose bengal solution is irradiated is light containing a wavelength of 537 nm. Examples of a device for irradiation with light include an LED light-emitting device that emits light containing a wavelength of 537 nm, and a lighting device that emits visible light. The light applied to the aqueous rose bengal solution may be natural light.

The illuminance of the light applied to the aqueous rose bengal solution or the light with which the aqueous rose bengal solution is irradiated is preferably not less than 500 lux as shown in Examples described later. If the illuminance of the light applied to the aqueous rose bengal solution is at least 500 lux, the new coronavirus SARS-CoV-2 can be inactivated within a short time. If the illuminance is less than 500 lux, the speed of inactivation of the new coronavirus SARS-CoV-2 cannot be considered sufficient for practical use. If the illuminance is increased, the speed of inactivation of the new coronavirus SARS-CoV-2 can be increased. However, judging from economic efficiency, efficiency, practicality, and the Examples described later, the upper limit of the illuminance is about 8000 lux.

A method for bringing the aqueous rose bengal solution and the air containing the new coronavirus SARS-CoV-2 into contact with each other in a state where the light is applied to the aqueous rose bengal solution is not particularly limited, but when the contact between the new coronavirus SARS-CoV-2 and singlet oxygen is considered, a large gas-liquid contact area is preferable.

One specific method is to blow the air containing the new coronavirus SARS-CoV-2 into the aqueous rose bengal solution to which the light is applied (first method). In the first method, in the aqueous rose bengal solution, the dissolved oxygen in the aqueous rose bengal solution and the oxygen in the blown air are excited to generate singlet oxygen, and the singlet oxygen inactivates the new coronavirus SARS-CoV-2.

In the case of the first method, even if the new coronavirus SARS-CoV-2 is not immediately inactivated by the singlet oxygen, the new coronavirus SARS-CoV-2 is trapped in the aqueous rose bengal solution. In addition, the new coronavirus SARS-CoV-2 adhering to dust, etc., in the air is also taken into the aqueous rose bengal solution together with the dust, etc.

As described above, in the case of the first method, the new coronavirus SARS-CoV-2 is inactivated or trapped in the aqueous rose bengal solution, so that purified air can be discharged. The new coronavirus SARS-CoV-2 trapped in the aqueous rose bengal solution is then inactivated by singlet oxygen.

The first method is preferably a method in which the air containing the new coronavirus SARS-CoV-2 and the aqueous rose bengal solution are brought into intense contact with each other such that the gas-liquid contact area is increased. One specific method is to provide an air blowing nozzle or air blowing hole such that the nozzle or hole is immersed in the aqueous rose bengal solution in a state where the light is applied to the aqueous rose bengal solution, and blow the air containing the new coronavirus SARS-CoV-2 into the aqueous rose bengal solution therethrough.

Examples of a second method include a method of spraying the aqueous rose bengal solution into the air containing the new coronavirus SARS-CoV-2 in a state where light is applied to the air, a method of spraying the aqueous rose bengal solution upward like a fountain, and a method of spraying the aqueous rose bengal solution downward like a shower.

As compared to the first method, the second method is more easily embodied into a device, and has a smaller pressure loss when sending the air containing the new coronavirus SARS-CoV-2. On the other hand, the first method is more efficient than the second method since the new coronavirus SARS-CoV-2 is easily trapped in the aqueous rose bengal solution.

The two specific methods have been described above as the new coronavirus inactivation method of the present invention, but have different characteristics, and thus may be selected and used according to the location of use, etc., as appropriate. Also, it is preferable to take advantage of the characteristics of the new coronavirus inactivation method when embodying the method into a device.

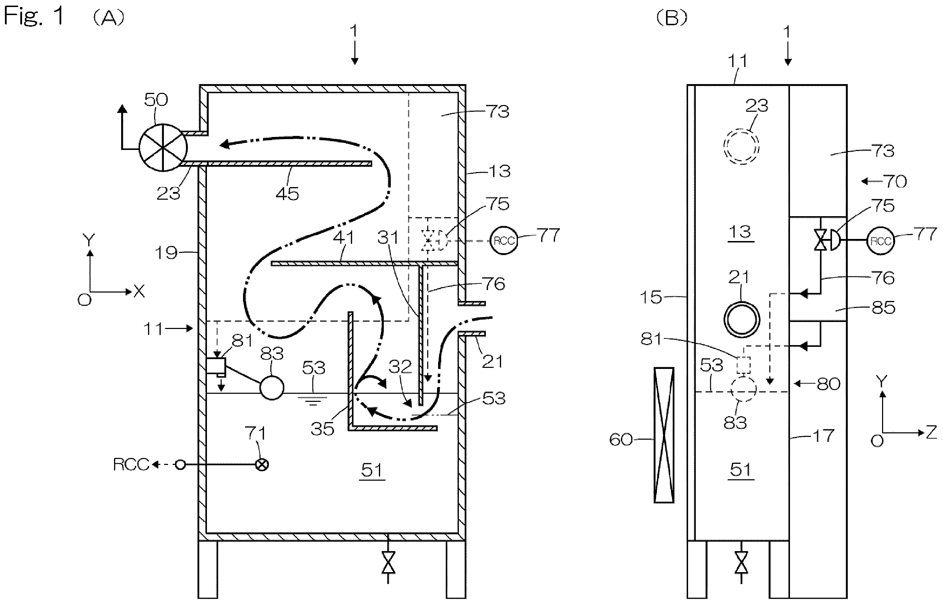

Fig. 1 illustrates the configuration of a new coronavirus inactivation device 1 of a first embodiment of the present invention, (A) is a cross-sectional view from the front, and (B) is a side view. In Fig. 1(A), a thick alternate long and two short dashes line schematically indicates the flow of the air for ease of understanding.

In Fig. 1, X, Y, and Z axes correspond to the X, Y, and Z axes of three-dimensional orthogonal coordinates. In the present embodiment, upward and up correspond to a Y-axis positive direction, downward and down correspond to a Y-axis negative direction, right corresponds to an X-axis positive direction, left corresponds to an X-axis negative direction, the far or back side corresponds to a Z-axis positive direction, the near or front side corresponds to a Z-axis negative direction, the vertical direction is parallel to the Y-axis direction in Fig. 1, and the horizontal direction is parallel to the X-axis direction in Fig. 1. In the present embodiment, a description will be given with the front side as front, the back side as rear, an air inlet side as upstream, and an air outlet side as downstream. These apply to the other embodiments.

The new coronavirus inactivation device 1 is a jet type device that includes a jet hole (air blowing hole) 32 and blows the air containing the new coronavirus SARS-CoV-2 into a stored aqueous rose bengal solution 51 through the jet hole 32 to bring the aqueous rose bengal solution 51 and the air containing the new coronavirus SARS-CoV-2 into contact with each other.

The new coronavirus inactivation device 1 includes a rectangular parallelepiped-shaped casing 11 which is narrow in width (Z direction), long in length (X direction), and high in height (Y direction), an air inlet 21 is provided in a right side wall 13 of the casing 11, and an air outlet 23 is provided in a left side wall 19 of the casing 11. The position in the height direction of the air inlet 21 is substantially at the center of the casing 11, and the position in the height direction of the air outlet 23 is at an upper portion of the casing 11. A front wall 15 of the casing 11 is a colorless transparent resin plate that can transmit light.

A first baffle plate 31 is mounted vertically at a portion near the air inlet 21 in the casing 11. The first baffle plate 31 is fixed at an upper end thereof to a third baffle plate 41, and is immersed at a lower portion thereof in the aqueous rose bengal solution 51. The first baffle plate 31 is mounted such that the front surface thereof is in contact with the front wall 15 and the back surface thereof is in contact with a rear wall 17.

An L-shaped second baffle plate 35 is mounted downstream of the first baffle plate 31 and below the third baffle plate 41. The second baffle plate 35 has a space between an upper end thereof and the third baffle plate 41, and is mounted such that a lower portion from the center thereof is immersed in the aqueous rose bengal solution 51. The second baffle plate 35 is mounted such that the front surface and the rear surface thereof are in contact with the casing 11. The space between the second baffle plate 35 and the third baffle plate 41 is an air flow path. In this inactivation device 1, the jet hole 32 is formed by the lower end of the first baffle plate 31 and the second baffle plate 35 located below the first baffle plate 31.

The third baffle plate 41 supports the first baffle plate 31 and forms an air flow path in the casing 11. Furthermore, the third baffle plate 41 serves as an inertial dust collector that allows the air containing droplets/mist (hereinafter, referred to as liquid particles) of the aqueous rose bengal solution 51 to collide thereagainst to remove the liquid particles in the air.

The third baffle plate 41 is horizontally mounted above the air inlet 21 in the casing 11. The third baffle plate 41 is mounted such that a right end thereof is in contact with the right side wall 13 of the casing 11, a front-side end portion thereof is in contact with the front wall 15 of the casing 11, and a rear-side end portion thereof is in contact with the rear wall 17 of the casing 11.

A fourth baffle plate 45 is installed below the air outlet 23 and above the third baffle plate 41 in the casing 11. The fourth baffle plate 45 is fixed at a left end thereof to the left side wall 19 of the casing 11. The fourth baffle plate 45 is mounted such that a front-side end portion thereof is in contact with the front wall 15 of the casing 11 and a rear-side end portion thereof is in contact with the rear wall 17 of the casing 11. Such a fourth baffle plate 45 forms an air flow path together with the third baffle plate 41, and serves as an inertial dust collector that allows the air containing liquid particles of the aqueous rose bengal solution 51 to collide thereagainst to remove the liquid particles.

A suction fan 50 is mounted at the air outlet 23. By putting the suction fan 50 into operation, the pressure of the casing 11 becomes negative, and the air containing the new coronavirus SARS-CoV-2 is sucked into the casing 11 through the air inlet 21. In the present embodiment, the suction fan 50 is mounted at the air outlet 23, but a forced-draft fan may be mounted at the air inlet 21 instead of the suction fan 50.

A lower portion in the casing 11 is a storage portion for the aqueous rose bengal solution 51, and the aqueous rose bengal solution 51 is stored therein. The aqueous rose bengal solution 51 is an aqueous rose bengal solution having a specific concentration in the range of not less than 10 µM and not greater than 50 µM. When the device is stopped, a water surface 53 of the aqueous rose bengal solution 51 is slightly higher than the lower end of the first baffle plate 31, and the jet hole 32 is submerged in the aqueous rose bengal solution 51.

A lighting device 60 for irradiating the aqueous rose bengal solution 51 with light is installed on the outer front surface of the casing 11. The lighting device 60 can irradiate the aqueous rose bengal solution 51 with light having a wavelength that excites rose bengal and having at least not less than 500 lux and not greater than 8000 lux. The casing 11 of the present embodiment has a narrow width (depth), so that the installation of a lighting device on the rear side thereof can be omitted. If necessary, light may also be applied from the rear side of the casing 11.

The new coronavirus inactivation device 1 of the present embodiment includes a concentration adjusting device 70 which adjusts the concentration of the aqueous rose bengal solution 51 into a preset range. The concentration adjusting device 70 includes a sensor 71 which detects the concentration of the aqueous rose bengal solution 51, a storage tank 73 which stores therein a high-concentration aqueous rose bengal solution, a concentration adjusting valve 75 which is mounted on the storage tank 73, and a concentration adjustor 77 which controls the concentration adjusting valve 75.

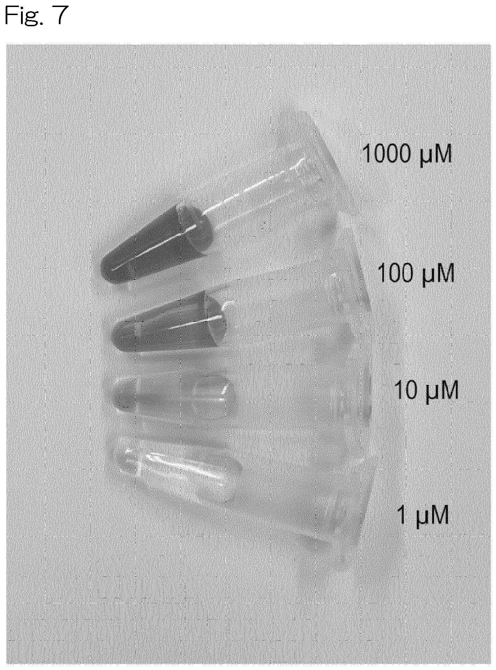

The sensor 71 directly detects the color of the aqueous rose bengal solution 51. When the concentration of an aqueous rose bengal solution is decreased, the color of the aqueous rose bengal solution is lighter and closer to white, and conversely when the concentration of an aqueous rose bengal solution is increased, the color of the aqueous rose bengal solution is darker and closer to reddish purple (see Fig. 7). Since there is a correlation between the concentration and the color of an aqueous rose bengal solution as described above, the concentration of an aqueous rose bengal solution can be obtained by measuring the color of the aqueous rose bengal solution. The sensor 71 may be a colorimeter.

The sensor 71 is installed on a side opposite to the jet hole 32 across the second baffle plate 35 in order to avoid air bubbles and liquid turbulence in the aqueous rose bengal solution 51. In the present embodiment, the concentration of the aqueous rose bengal solution 51 is detected from the color of the aqueous rose bengal solution 51, but the method for detecting the concentration of the aqueous rose bengal solution 51 and the sensor used are not limited thereto.

The storage tank 73 is fixed to an outer wall surface upper portion of the rear surface of the casing 11. When the storage tank 73 is installed at such a high position, the high-concentration aqueous rose bengal solution can be supplied by using gravity. In the storage tank 73, an aqueous rose bengal solution having a high concentration, e.g., 1 mM, is contained. It is needless to say that the high-concentration aqueous rose bengal solution has a higher concentration than the aqueous rose bengal solution 51 in the casing 11.

The concentration adjusting valve 75 is mounted on a bottom portion of the storage tank 73, and includes, at a discharge side thereof, a supply pipe 76 through which the high-concentration aqueous rose bengal solution is introduced into the casing 11. The supply pipe 76 supplies the high-concentration aqueous rose bengal solution to the upstream side of the jet hole 32. The high-concentration aqueous rose bengal solution supplied thus is guided to the jet hole 32, and is vigorously mixed with the aqueous rose bengal solution 51, and the mixture is homogenized within a short time.

The concentration adjustor 77 is connected to the sensor 71, receives a signal from the sensor 71, and controls the concentration adjusting valve 75 such that the aqueous rose bengal solution 51 in the casing 11 has a set concentration.

The new coronavirus inactivation device 1 also includes a water level adjusting device 80 which keeps the water level of the aqueous rose bengal solution 51 at a set water level. The water level adjusting device 80 includes a ball tap 81, which is a water level adjusting valve, and a storage tank 85 which supplies water to the ball tap 81.

The ball tap 81 is a known ball tap that includes a float 83 and automatically adjusts a valve opening degree thereof according to the water level. The float 83 is installed on the side opposite to the jet hole 32 across the second baffle plate 35, where there is less water level disturbance. The storage tank 85 is fixed to the outer wall surface upper portion of the rear surface of the casing 11. In the present embodiment, the ball tap 81 is used as the water level adjusting device 80, but the water level adjusting device 80 is not limited thereto. The water level adjusting device 80 may be composed of a water level gauge, a water level adjusting valve, and a water level adjuster, and a tap water supply line may be used as a water supply source instead of the storage tank 85.

The operation of the new coronavirus inactivation device 1 will be described. When the suction fan 50 is put into operation, the air containing the new coronavirus SARS-CoV-2 is sucked through the air inlet 21. The sucked air containing the new coronavirus SARS-CoV-2 is blown into the aqueous rose bengal solution 51 through the jet hole 32.

When the air flows into the jet hole 32, the water surface 53 moves up and down due to the pressure balance, and when the air passes through the jet hole 32 at a high speed, the aqueous rose bengal solution 51 around the jet hole 32 is greatly disturbed. The air ejected from the jet hole 32 also disturbs the aqueous rose bengal solution 51 around the first baffle plate 31, and further collides against the second baffle plate 35 together with a part of the aqueous rose bengal solution 51, thereby vigorously disturbing the aqueous rose bengal solution 51 around the second baffle plate 35.

Due to these, the air containing the new coronavirus SARS-CoV-2 is entrained into the aqueous rose bengal solution 51, and is also taken into the aqueous rose bengal solution 51 as air bubbles. At this time, the aqueous rose bengal solution 51 is irradiated with the light from the lighting device 60, so that the new coronavirus SARS-CoV-2 taken into the aqueous rose bengal solution 51 together with the air is inactivated by generated singlet oxygen.

The new coronavirus SARS-CoV-2 is inactivated by the aqueous rose bengal solution 51, and the purified air from which the new coronavirus SARS-CoV-2 has been taken into the aqueous rose bengal solution 51 collides against the third baffle plate 41 and the fourth baffle plate 45 to thereby separate the accompanying liquid particles, and is discharged through the suction fan 50 provided at the air outlet 23.

As described above, in the new coronavirus inactivation device 1, the air containing the new coronavirus SARS-CoV-2 is jetted into the aqueous rose bengal solution 51, and the air containing the new coronavirus SARS-CoV-2 and the aqueous rose bengal solution 51 come into intense contact with each other in a state where light is applied thereto, so that the new coronavirus SARS-CoV-2 is inactivated within a short time. In addition, the new coronavirus SARS-CoV-2 is taken into the aqueous rose bengal solution 51, so that the air containing the new coronavirus SARS-CoV-2 can be efficiently and quickly purified.

The rose bengal used in the new coronavirus inactivation device 1 is safe, as can be seen from the fact that rose bengal is used as a food coloring agent. In addition, the singlet oxygen which inactivates the new coronavirus SARS-CoV-2 disappears within a very short time and is therefore safe. As can be seen from these, the new coronavirus inactivation device 1 can be considered as a highly safe device.

Furthermore, since the new coronavirus inactivation device 1 includes the concentration adjusting device 70 which keeps the concentration of the aqueous rose bengal solution 51 within a set range and the water level adjusting device 80 which keeps the water level of the aqueous rose bengal solution 51 constant, the new coronavirus inactivation device 1 is capable of continuously operating over a long period of time.

Moreover, since the configuration of the new coronavirus inactivation device 1 is simple, the new coronavirus inactivation device 1 is easily downsized or upsized. The new coronavirus inactivation device 1 is also inexpensive in running cost.

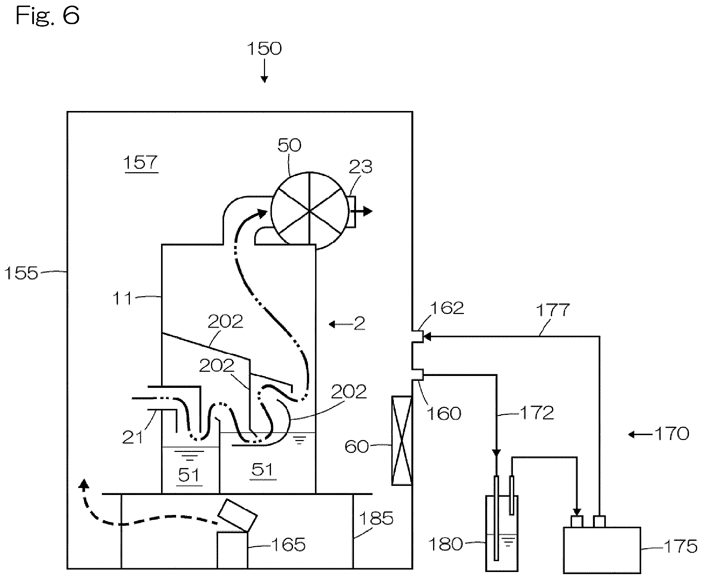

The jet type new coronavirus inactivation device 1 is not limited to the device shown in Fig. 1. In the new coronavirus inactivation device 1, a jet nozzle may be provided instead of the jet hole 32. A new coronavirus inactivation device 2 shown in Fig. 6 and used in Example 1 described later is also a jet type, and such a device is suitable for use as a new coronavirus inactivation device.

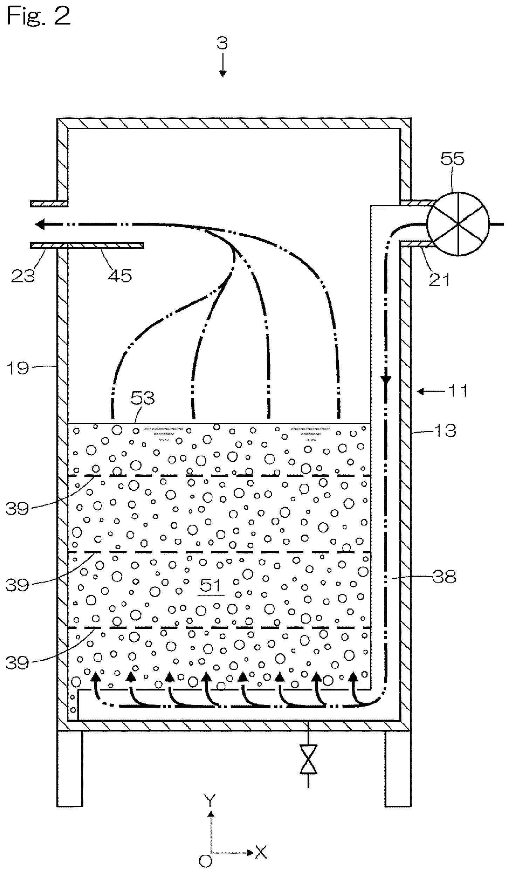

Fig. 2 illustrates the configuration of a new coronavirus inactivation device 3 of a second embodiment of the present invention. In the drawing, a thick alternate long and two short dashes line schematically indicates the flow of the air for ease of understanding. In Fig. 2, a lighting device 60, a concentration adjusting device 70, and a water level adjusting device 80 are not shown, but the new coronavirus inactivation device 3 also includes a lighting device 60, a concentration adjusting device 70, and a water level adjusting device 80 which are the same as in the new coronavirus inactivation device 1. The same components as in the new coronavirus inactivation device 1 of the first embodiment of the present invention shown in Fig. 1 are denoted by the same reference numerals, and the description thereof is omitted.

The new coronavirus inactivation device 3 includes an air supply pipe 38 through which the air containing the new coronavirus SARS-CoV-2 is sent to a lower portion of the aqueous rose bengal solution 51. The new coronavirus inactivation device 3 also includes dispersion plates 39 for dispersing the air sent through the air supply pipe 38, in the aqueous rose bengal solution 51.

The air containing the new coronavirus SARS-CoV-2 and sent from a forced-draft fan 55 which is provided at the air inlet 21 is blown into the aqueous rose bengal solution 51 through the air supply pipe 38, is further dispersed by the dispersion plates 39, and is inactivated by the action of singlet oxygen. The new coronavirus inactivation device 3 has commonalities with the new coronavirus inactivation device 1 in that the air containing the new coronavirus SARS-CoV-2 is blown into the aqueous rose bengal solution 51 and the new coronavirus SARS-CoV-2 is inactivated.

In the new coronavirus inactivation device 3, the concentration of the aqueous rose bengal solution 51 and the illuminance of light with which the aqueous rose bengal solution 51 is irradiated are the same as in the new coronavirus inactivation device 1, and the action and the effects of the new coronavirus inactivation device 3 and the procedure for inactivating the new coronavirus SARS-CoV-2 are also basically the same as in the new coronavirus inactivation device 1.

The new coronavirus inactivation devices 1, 2, and 3 shown in Fig. 1, Fig. 2, and Fig. 6 can each be considered as a device that embodies the first method of the new coronavirus inactivation method of the present invention. Next, new coronavirus inactivation devices 4, 5, 6, and 7 which embody the second method of the new coronavirus inactivation method of the present invention will be described.

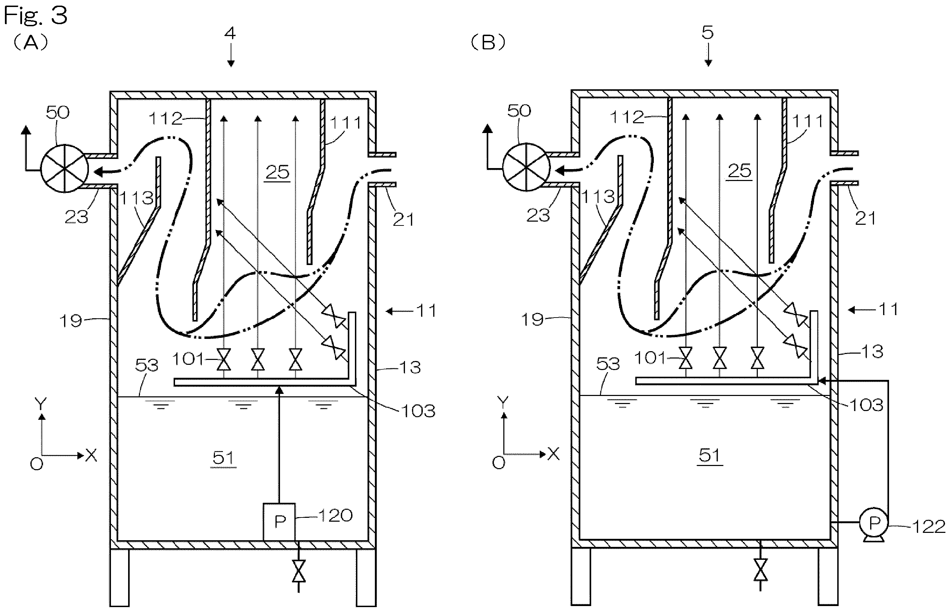

Fig. 3 illustrates the configurations of new coronavirus inactivation devices 4 and 5 of a third embodiment of the present invention. In the drawing, a thick alternate long and two short dashes line schematically indicates the flow of the air for ease of understanding. In Fig. 3, a lighting device 60, a concentration adjusting device 70, and a water level adjusting device 80 are not shown, but the new coronavirus inactivation devices 4 and 5 each also include a lighting device 60, a concentration adjusting device 70, and a water level adjusting device 80 which are the same as in the new coronavirus inactivation device 1. The same components as in the new coronavirus inactivation device 1 of the first embodiment of the present invention shown in Fig. 1 are denoted by the same reference numerals, and the description thereof is omitted.

The new coronavirus inactivation device 4 shown in Fig. 3(A) injects the aqueous rose bengal solution 51 stored in the casing 11, toward a gas phase portion 25 in the casing 11 through an injection nozzle 101, brings the air containing the new coronavirus SARS-CoV-2 and the injected aqueous rose bengal solution 51 into contact with each other in the gas phase portion 25, and inactivates the new coronavirus SARS-CoV-2 by generated singlet oxygen.

Three baffle plates 111, 112, and 113 are mounted in the gas phase portion 25 from upstream toward downstream. The three baffle plates 111, 112, and 113 form an air flow path. Furthermore, the baffle plate 111 and the baffle plate 112 form a region where the air containing the new coronavirus SARS-CoV-2 and flowing in from the air inlet 21 and the injected aqueous rose bengal solution 51 are brought into contact with each other, and the baffle plates 112 and 113 prevent the injected aqueous rose bengal solution 51 from being carried to the air outlet 23.

As the injection nozzle 101, a plurality of nozzles are mounted on an aqueous rose bengal solution supply pipe 103 installed horizontally and vertically, and inject the aqueous rose bengal solution at a high speed toward a section formed by the baffle plate 111 and the baffle plate 112. The aqueous rose bengal solution injected at the high speed collides against the baffle plates 111 and 112 and the ceiling surface of the casing 11 to change its direction, and finally falls downward. In this process, the injected aqueous rose bengal solution is subdivided or made into small droplets or mist, thereby increasing the gas-liquid contact area.

A submersible pump 120 submerged in the aqueous rose bengal solution 51 is connected to the aqueous rose bengal solution supply pipe 103, and the aqueous rose bengal solution 51 is supplied through the submersible pump 120.

The lighting device 60 installed in the new coronavirus inactivation device 4 applies light toward the gas phase portion 25. The wavelength and the illuminance of the light are the same as in the new coronavirus inactivation device 1. In addition, the concentration of the aqueous rose bengal solution 51 in the new coronavirus inactivation device 4 is also the same as in the new coronavirus inactivation device 1. In the new coronavirus inactivation device 4, the suction fan 50 is provided at the air outlet 23 to suck the air into the casing 11, but a forced-draft fan may be installed at the air inlet 21 to push the air into the casing 11. This point is the same as in the new coronavirus inactivation device 1 of the first embodiment.

The new coronavirus inactivation device 5 shown in Fig. 3(B) has the same basic configuration, action, and effects as the new coronavirus inactivation device 4. The difference from the new coronavirus inactivation device 4 is that the pump that supplies the aqueous rose bengal solution 51 to the aqueous rose bengal solution supply pipe 103 is an external pump 122.

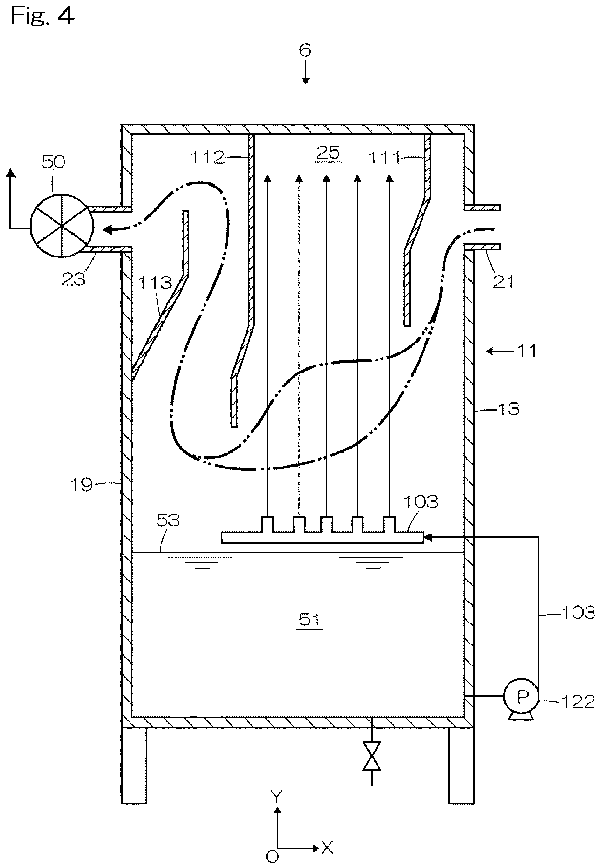

Fig. 4 illustrates the configuration of a new coronavirus inactivation device 6 which is a fourth embodiment of the present invention. In the drawing, a thick alternate long and two short dashes line schematically indicates the flow of the air for ease of understanding. In Fig. 4, a lighting device 60, a concentration adjusting device 70, and a water level adjusting device 80 are not shown, but the new coronavirus inactivation device 6 also includes a lighting device 60, a concentration adjusting device 70, and a water level adjusting device 80 which are the same as in the new coronavirus inactivation device 1. The same components as in the new coronavirus inactivation device 1, which is the first embodiment of the present invention shown in Fig. 1, and the new coronavirus inactivation device 5, which is the third embodiment, are denoted by the same reference numerals, and the description thereof is omitted.

The new coronavirus inactivation device 6 is a device similar in structure to the new coronavirus inactivation device 5, brings the air containing the new coronavirus SARS-CoV-2 and the injected aqueous rose bengal solution 51 into contact with each other in the gas phase portion 25 in the casing 11, and inactivates the new coronavirus SARS-CoV-2 by generated singlet oxygen.

The difference between the new coronavirus inactivation device 6 and the new coronavirus inactivation device 5 is the procedure for injecting the aqueous rose bengal solution 51 to the gas phase portion 25. While the new coronavirus inactivation device 5 injects the aqueous rose bengal solution at a high speed toward the section formed by the baffle plate 111 and the baffle plate 112 using the injection nozzles 101, the new coronavirus inactivation device 6 injects the aqueous rose bengal solution like a fountain.

The other components of the new coronavirus inactivation device 6 are the same as those of the new coronavirus inactivation device 1 of the first embodiment and the new coronavirus inactivation device 5 of the third embodiment, and thus the description thereof is omitted.

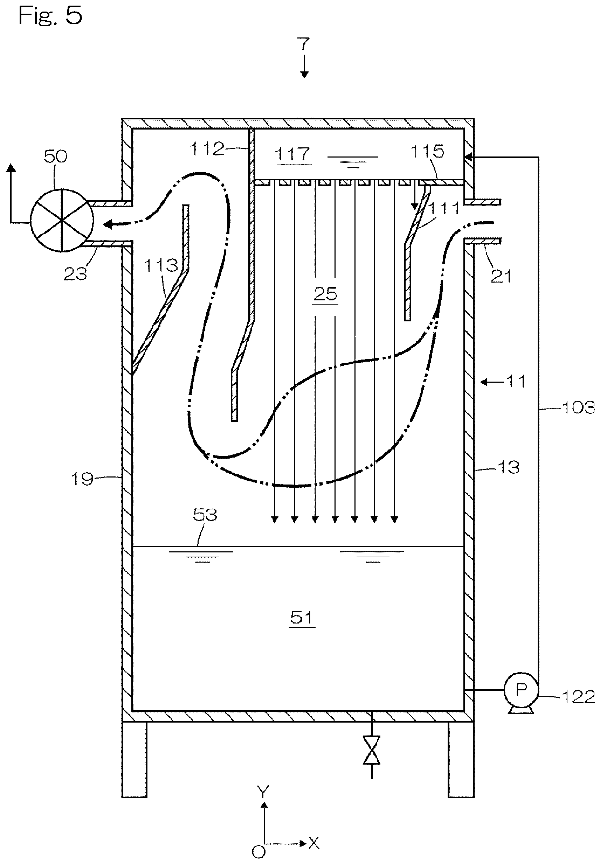

Fig. 5 illustrates the configuration of a new coronavirus inactivation device 7 which is a fifth embodiment of the present invention. In the drawing, a thick alternate long and two short dashes line schematically indicates the flow of the air for ease of understanding. In Fig. 5, a lighting device 60, a concentration adjusting device 70, and a water level adjusting device 80 are not shown, but the new coronavirus inactivation device 7 also includes a lighting device 60, a concentration adjusting device 70, and a water level adjusting device 80 which are the same as in the new coronavirus inactivation device 1. The same components as in the new coronavirus inactivation device 1 of the first embodiment of the present invention shown in Fig. 1 and the new coronavirus inactivation device 6 of the fourth embodiment are denoted by the same reference numerals, and the description thereof is omitted.

The new coronavirus inactivation device 7 is a device similar in structure to the new coronavirus inactivation device 6, brings the air containing the new coronavirus SARS-CoV-2 and the injected aqueous rose bengal solution 51 into contact with each other in the gas phase portion 25 in the casing 11, and inactivates the new coronavirus SARS-CoV-2 by generated singlet oxygen.

The difference between the new coronavirus inactivation device 7 and the new coronavirus inactivation device 6 is the procedure for supplying the aqueous rose bengal solution 51 to the gas phase portion 25. In the new coronavirus inactivation device 7, a perforated plate 115 is mounted below the ceiling surface of the casing 11, and a supply chamber 117 for an aqueous rose bengal solution is provided. The supply chamber 117 is connected to the pump 122 via the aqueous rose bengal solution supply pipe 103. The aqueous rose bengal solution sent to the supply chamber 117 falls like a shower from the perforated plate 115.

The other components of the new coronavirus inactivation device 7 are the same as those of the new coronavirus inactivation device 1 of the first embodiment and the new coronavirus inactivation device 6 of the fourth embodiment, and thus the description thereof is omitted.

The new coronavirus inactivation devices 4, 5, 6, and 7, which embody the second method of the new coronavirus inactivation method, also basically have the same action and effects as the new coronavirus inactivation devices 1, 2, and 3, which embody the first method of the new coronavirus inactivation method. The new coronavirus inactivation devices 4, 5, 6, and 7 also have commonalities with the new coronavirus inactivation devices 1, 2, and 3 in that the devices efficiently and quickly purify the air containing the new coronavirus SARS-CoV-2, are highly safe, are capable of continuously operating over a long period of time, are easily downsized or upsized, and are inexpensive in running cost.

Although the new coronavirus inactivation device according to the present invention and the new coronavirus inactivation method according to the present invention have been described above by means of the new coronavirus inactivation devices of the first to fifth embodiments, the new coronavirus inactivation method and device according to the present invention are not limited to the above embodiments, and may be modified without departing from the gist of the invention, and the modified ones can be used.

In the above embodiments, the high-concentration aqueous rose bengal solution is supplied to adjust the concentration of the aqueous rose bengal solution 51, but rose bengal powder may be supplied instead of the high-concentration aqueous rose bengal solution. In the case where rose bengal powder is used to adjust the concentration of the aqueous rose bengal solution 51, a device suitable for supplying the powder may be used.

In each of the new coronavirus inactivation devices of the above embodiments, the baffle plate is provided near the air outlet 23 to separate liquid particles of the aqueous rose bengal solution contained in the purified air, but a demister, a filter, or an electric dust collector may be provided instead of or together with the baffle plate. In the new coronavirus inactivation device according to the present invention, the number, the shapes, and the installation manners of the baffle plates and the shape of the casing are not limited to those in the above embodiments.

Although the present invention has been fully described in connection with the preferred embodiments thereof with reference to the accompanying drawings which are used only for the purpose of illustration, those skilled in the art will readily conceive numerous changes and modifications within the framework of obviousness upon the reading of the specification herein presented of the present invention. Accordingly, such changes and modifications are, unless they depart from the scope of the present invention as delivered from the claims annexed hereto, to be construed as included therein.

### EXAMPLES

### Example 1

A new coronavirus inactivation test was conducted using a jet type new coronavirus inactivation device. Fig. 6 schematically shows the configuration of a test apparatus. In Fig. 6, a thick alternate long and two short dashes line schematically indicates the flow of the air, and a thick broken line indicates the flow of the new coronavirus SARS-CoV-2 released from a nebulizer. The same components as in the new coronavirus inactivation device 1 of the first embodiment of the present invention shown in Fig. 1 are denoted by the same reference numerals, and the description thereof is omitted.

The test apparatus 150 consists mainly of a chamber 155 in which the new coronavirus inactivation device 2 is housed, a nebulizer 165 which releases the new coronavirus SARS-CoV-2 into the chamber 155, and a sampling device 170 which samples the air in the chamber 155.

The chamber 155 is composed of a transparent resin material and forms a sealed space therein. An interior space 157 has a size with a length of 900 mm, a width of 740 mm, and a height of 1040 mm, and has an internal capacity of 693 L. The chamber 155 is provided with a sampling nozzle 160 and a return nozzle 162 for returning the air returned from the sampling device 170.

The nebulizer 165 is installed below a mount 185 on which the new coronavirus inactivation device 2 is placed, and releases the new coronavirus SARS-CoV-2 into the interior space 157. The spraying ability of the nebulizer 165 is 0.25 to 0.90 mL/min.

The sampling device 170 includes a suction pump 175 which sucks the air in the interior space 157, and an impinger 180 which collects the new coronavirus SARS-CoV-2 in the sucked air into a collection solution. The impinger 180 is mounted on a sampling tube 172 which connects the sampling nozzle 160 and the suction pump 175. The air exiting the impinger 180 is returned into the chamber 155 through a return tube 177 which connects the suction pump 175 and the return nozzle 162.

The new coronavirus inactivation device 2 is placed on the mount 185 installed in the chamber 155. The new coronavirus inactivation device 2 is a jet type device that blows the air containing the new coronavirus SARS-CoV-2 into the aqueous rose bengal solution 51.

The new coronavirus inactivation device 2 includes a plurality of baffle plates 202 which form an air flow path in the casing 11, and the air containing the new coronavirus SARS-CoV-2 and sucked from the air inlet 21 is guided to the baffle plates 202 and blown into the aqueous rose bengal solution 51. The aqueous rose bengal solution 51 is irradiated with the light from the lighting device 60, and the new coronavirus SARS-CoV-2 is inactivated by generated singlet oxygen and discharged through the air outlet 23. The volume of the aqueous rose bengal solution 51 is 2.5 L on the left side and 6.5 L on the right side.

Since the air inlet 21 and the air outlet 23 of the new coronavirus inactivation device 2 are inside the chamber 155, the new coronavirus inactivation device 2 sucks the air in the chamber 155, inactivates the air, and discharges the purified air into the chamber 155. In other words, the air in the chamber 155 can be considered to circulate in the chamber 155 while being purified through the new coronavirus inactivation device 2. The airflow rate of the suction fan 50 of the new coronavirus inactivation device 2 is 4.14 m³/min.

The test procedure will be described below. The nebulizer 165 was operated for 5 minutes and then stopped, and the suction fan 50 of the new coronavirus inactivation device 2 was put into operation to start the test. Thereafter, as appropriate, the air in the interior space 157 was guided to the impinger 180 via the sampling device 170, and the new coronavirus SARS-CoV-2 was collected into an aqueous solution. The viral genome RN copy number and the virus infectivity titer in the obtained sample were measured. At this time, the concentration of the aqueous rose bengal solution 51 was 10 µM, and the illuminance was 7170 lux.

As a result of the test, the air in the interior space 157 after operating the nebulizer 165 for 5 minutes had approximately 2×10⁶ to 7×10⁷ copies per L of the air and viruses of 2 to 6×10³ TCID50 in infectivity titer.

When the new coronavirus inactivation device 2 was put into operation, the virus infectivity titer was decreased to about 1/10 in 2 to 3 minutes, and was no longer detected in 6 to 7 minutes. The virus infectivity titer was also not detected in the aqueous rose bengal solution 51 stored in the new coronavirus inactivation device 2, and the viruses were all inactivated.

### Example 2

In a safety cabinet, 25 mL of distilled water was put in a glass test tube, 250 µL of the new coronavirus was added, and the tube was left at room temperature for 15 minutes. Then, rose bengal was added such that the concentration thereof was 10 µM, and 10-fold serial dilution was performed immediately. The illuminance at this time was 600 lux. As a result of measuring a virus infectivity titer by the TCID₅₀ method, nCoV was 63 TCID₅₀/ml which is the detection limit. In the case of a comparative example in which rose bengal was not added, even after the new coronavirus was added and kept for 15 minutes under an illuminance of 600 lux, nCoV was 1.1×10⁶. This indicates that the new coronavirus is inactivated within a very short time (within a few seconds) when the new coronavirus comes into contact with the aqueous rose bengal solution and light having an illuminance of 600 lux is applied thereto.

### [Reference Numerals]

- 1, 2, 3, 4, 5, 6, 7: new coronavirus inactivation device
- 11: casing
- 21: air inlet
- 23: air outlet
- 25: gas phase portion
- 32: jet hole
- 50: suction fan
- 51: aqueous rose bengal solution
- 53: water surface
- 60: lighting device
- 70: concentration adjusting device
- 71: sensor
- 73: storage tank
- 75: concentration adjusting valve
- 77: concentration adjustor
- 80: water level adjusting device
- 81: ball tap
- 85: storage tank
- 101: injection nozzle
- 150: test apparatus
- 155: chamber
- 165: nebulizer
- 170: sampling device
- 180: impinger

## Claims

1. A method for inactivating a new coronavirus, the method comprising:
bringing an aqueous rose bengal solution and air containing new coronavirus SARS-CoV-2 into contact with each other in a state where light is applied to the aqueous rose bengal solution to generate singlet oxygen; and
inactivating the new coronavirus SARS-CoV-2 by the generated singlet oxygen; wherein
the aqueous rose bengal solution has a concentration of not less than 10 µM and not greater than 50 µM, and
the light applied to the aqueous rose bengal solution has an illuminance of not less than 500 lux and not greater than 8000 lux.

2. A new coronavirus inactivation device for inactivating new coronavirus SARS-CoV-2 by singlet oxygen, the new coronavirus inactivation device comprising:
a contactor configured to bring an aqueous rose bengal solution and air containing the new coronavirus SARS-CoV-2 into contact with each other in a state where light is applied to the aqueous rose bengal solution, wherein
the aqueous rose bengal solution has a concentration of not less than 10 µM and not greater than 50 µM, and
the light applied to the aqueous rose bengal solution has an illuminance of not less than 500 lux and not greater than 8000 lux.

3. The new coronavirus inactivation device as claimed in claim 2, wherein the contactor is configured to blow the air containing the new coronavirus SARS-CoV-2 into the aqueous rose bengal solution to which the light is applied.

4. The new coronavirus inactivation device as claimed in claim 3, wherein the contactor configured to blow the air includes a jet nozzle or jet hole through which the air containing the new coronavirus SARS-CoV-2 is blown into the aqueous rose bengal solution to which the light is applied.

5. The new coronavirus inactivation device as claimed in any one of claims 2 to 4, further comprising a concentration adjusting device including a sensor configured to detect the concentration of the aqueous rose bengal solution and a supplier configured to supply rose bengal powder or a high-concentration aqueous rose bengal solution, the concentration adjusting device being configured to adjust the concentration of the aqueous rose bengal solution into a set range.

6. The new coronavirus inactivation device as claimed in claim 5, wherein the concentration adjusting device detects a color of the aqueous rose bengal solution by the sensor and adjusts the concentration of the aqueous rose bengal solution into the set range by using a correlation between the color and the concentration of the aqueous rose bengal solution.

7. The new coronavirus inactivation device as claimed in any one of claims 2 to 4, further comprising a water level adjusting device configured to keep a water level of the aqueous rose bengal solution at a set water level.
